# FASCICULE DE BREVET EUROPEEN

(11) **EP 2 375 979 B1**
(45) Date de publication et mention de la délivrance du brevet: **11.12.2019**
(21) Numéro de dépôt: 10706017.0
(22) Date de dépôt: 13.01.2010
(51) Int. Cl.: A61B 5/103, A61F 13/08

(54) **SYSTEME DE MESURE DE PRESSION D'INTERFACE**
SYSTEM ZUR MESSUNG EINES SCHNITTSTELLENDRUCKS
SYSTEM FOR MEASURING INTERFACE PRESSURE

(30) Priorité: 13.01.2009 FR 0950147
(43) Date de publication de la demande: 19.10.2011
(73) Titulaire: Urgo Recherche Innovation et Développement, 21300 Chenove (FR)
(72) Inventeur: LAMOISE, Michel, F-21110 Bessey Les Citeaux (FR); LECOMTE, Serge, F-21000 Dijon (FR); STEINBRUNN, Julien, F-21000 Dijon (FR)
(74) Mandataire: Hubert, Philippe
(86) Numéro de dépôt international: PCT/FR2010/050044
(87) Numéro de publication internationale: WO 2010/081989

(56) Documents cités:
- EP-A- 1 884 226
- WO-A-00/72797
- WO-A-2006/030405
- WO-A-2006/040109
- WO-A-2006/103422
- WO-A-2007/079777
- WO-A-2008/102308
- DE-A1-102007 020 247
- GB-A- 2 322 556
- GB-A- 2 439 750
- GB-A- 2 445 760
- US-A- 3 814 998
- US-A- 4 186 732
- US-A1- 2002 173 735
- US-A1- 2006 036 203
- US-B2- 7 270 642
- DEUTSCHES INSTITUT FÜR GÜTESICHERUNG UND KENNZEICHNUNG E.V.: "Medical Compression Hosiery Quality Assurance RAL-GZ 387/1" , [Online] 2008, XP002527249 RAL Extrait de l'Internet: URL:http://www.gzg-kompressionsstruempfe.d e/uploads/media/RAL_GZ_387_englische_Versi on.pdf> [extrait le 2009-05-11]

## Description

La présente invention concerne généralement un système de mesure de pression d'interface, en particulier d'une pression s'exerçant sur la peau. Elle trouve notamment application dans le domaine médical, en thérapie compressive.

La thérapie compressive est reconnue comme une technique efficace dans le traitement des maladies liées à l'insuffisance veineuse comme les thromboses, les oedèmes, les lymphoedèmes ou les ulcères de jambes.

Cette thérapie a recours à la pose de divers dispositifs de compression comme des chaussettes ou des bandages de compression, dont les caractéristiques variables d'élasticité permettent de choisir la fenêtre de pression thérapeutique à utiliser en fonction du patient et de ses pathologies.

Un dispositif de compression tel qu'un bandage doit répondre à deux objectifs :
- d'une part, appliquer une pression adéquate à la pose ;
- d'autre part, maintenir ce niveau de pression pendant plusieurs jours dans une plage donnée de pression thérapeutique, pour éviter de devoir repositionner le bandage ou en utiliser un nouveau.

Pour qu'un bandage soit efficace, il est donc non seulement nécessaire qu'il ait été correctement positionné mais également que le patient accepte de conserver ce bandage pendant plusieurs jours et de suivre les recommandations du personnel soignant, par exemple en réalisant des exercices physiques comme la marche dans le cadre de la prévention de la thrombose veineuse.

Pour une efficacité maximum, le système de compression doit être conservé de 5 à 7 jours et appliquer une pression décroissant graduellement en remontant le long de la jambe, le gradient de pression en résultant permettant de forcer le sang à remonter vers le haut et à ne pas stagner. A titre d'exemple, la pression appliquée peut varier entre une valeur haute de l'ordre de 30 à 40 mm de mercure au niveau de la malléole de la cheville et une valeur basse de l'ordre de 15 à 25 mm de mercure au niveau de la partie supérieure du mollet proche du genou.

Le maintien d'un différentiel de pression entre la cheville et le mollet est essentiel pour l'efficacité du traitement.

Cependant, les systèmes de compression actuellement commercialisés ne comportent pas de moyens permettant de mesurer ce différentiel de pression. De plus, ces systèmes ne permettent ni de connaitre la pression exacte appliquée ni de suivre son évolution au cours du temps et par conséquent de détecter les éventuels problèmes de sous ou surpression.

Le personnel soignant est donc incapable aujourd'hui de suivre ou vérifier l'observance par le patient de son traitement (exercice physique, port ou retrait du système de compression, etc.).

De ce fait l'utilisation de ces systèmes de compression connus est loin d'être optimale.

Il existe donc une forte demande, dans le cadre de la thérapie de compression, pour la mise au point d'un système de mesure de pression qui permette :
- de vérifier que la pression exercée par le système de compression est optimale non seulement au moment de la pose mais également au cours du temps ;
- de vérifier que le gradient de pression cheville-mollet reste optimal lors du traitement ;
- de détecter les éventuels problèmes relatifs à des sur ou sous pressions ;
- d'enregistrer les données au cours du temps afin de permettre au personnel soignant de vérifier l'observance du traitement par le patient.

Toutefois, la réalisation d'un tel système de mesure de pression se heurte à de nombreux obstacles techniques.

Une des principales difficultés à résoudre réside dans la mise au point d'un capteur permettant de mesurer de façon satisfaisante la pression d'interface exercée entre la peau et le système de compression.

En effet, ce capteur doit :
- avoir une grande sensibilité pour permettre la mesure de pressions faibles, comprises dans une plage variant de 1 à 120 mm de mercure, et en particulier de 5 à 60 mm de mercure, avec une grande précision de l'ordre de 1 mm de mercure ;
- être robuste pour pouvoir supporter une surcharge de 100 % (c'est-à-dire une pression de 250 mm de mercure) sans détérioration ;
- présenter une bonne linéarité;
- permettre d'effectuer des mesures fiables sur une longue durée (plusieurs jours) ;
- être apte à fonctionner aussi bien en mode statique (lorsque le patient est immobile) qu'en mode dynamique (lorsque le patient est en mouvement) et, par conséquent, il devra être capable de mesurer avec précision des pressions très faibles à des fréquences de l'ordre du Hertz, correspondant aux variations à enregistrer lors de la marche d'une personne, et pour des variations très rapides de la pression appliquée.

Ainsi, un capteur satisfaisant, dans l'application envisagée, doit au minimum présenter de bonnes propriétés de sensibilité et de linéarité, tant en mode statique qu'en mode dynamique, pour des mesures de pression très faibles.

Il a été déterminé que la linéarité sur la zone de mesure préférentielle (5-60 mm de mercure) doit être idéalement supérieure à 0,95 et de préférence encore supérieure ou égale à 0,98. En effet, si le capteur n'est pas linéaire, l'obtention de résultats facilement exploitables nécessitera l'utilisation d'un système électronique associé incorporant des dispositifs de correction pour rendre le signal électrique de sortie proportionnel aux variations des valeurs de pression mesurées. Le système en résultant sera plus complexe et son coût de revient plus élevé.

Pour permettre de mesurer avec précision des pressions très faibles pour des variations très rapides de la pression appliquée comme celles observées lors de la marche d'une personne, la surface du capteur doit avantageusement présenter une rémanence faible, c'est-à-dire qu'elle doit reprendre sa forme et son épaisseur initiale dans un délai très rapide lorsque la pression exercée a cessé pour effectuer une mesure correcte lors de l'application de la pression suivante.

Un capteur idéal doit en outre être souple et sa surface doit pouvoir s'adapter à la morphologie du patient, à la nature et à l'état de surface du membre (os, muscle, graisse) à traiter.

La surface du capteur doit encore s'adapter à la pression appliquée par le système de compression lequel n'est pas rigide. En effet, ce système est constitué d'une bande qui, en fonction de la forme et la dureté du membre à traiter, va déformer non seulement la peau mais également le capteur pour lui donner une forme courbe. Ce phénomène est connu sous le terme d'effet « Hamac » en référence à la forme prise par le capteur et la peau. Les forces de compression induites par le bandage sont en partie absorbées en raison de cet effet « Hamac » et, dans ces conditions, un capteur de pression habituel ne détecte quasiment plus rien.

C'est une des raisons pour lesquelles un système de mesure de pression comportant de nombreux capteurs est difficile à mettre au point dans la mesure où chaque capteur devrait avoir des propriétés spécifiques et le système électronique associé devrait être conçu pour gérer une multitude de signaux complexes.

Il est donc nécessaire, en raison de cet effet « Hamac » de disposer d'un capteur de pression dont les contraintes de courbure ont été minimisées pour assurer une bonne répartition des efforts exercés sur la surface de mesure.

Mais ceci doit être réalisé sans augmenter de façon importante l'épaisseur du capteur pour éviter d'altérer ses propriétés intrinsèques précédemment décrites.

En outre, un capteur trop épais ou trop rigide risque de blesser la peau du patient souvent fragile, en particulier dans le cadre du traitement d'un ulcère.

Il en résulte que la surface d'un capteur idéal doit posséder des propriétés spécifiques et contradictoires pour assurer à la fois un contact homogène et efficace sur l'ensemble de cette surface, grâce à une certaine rigidité visant à limiter l'effet « Hamac », et une résistance à l'écrasement et une souplesse suffisantes pour garantir un signal reproductible et linéaire sur une longue durée.

Comme on le comprend , la mise au point d'un système de mesure de pression répondant à l'ensemble des conditions définies ci-dessus est très complexe et c'est une des raisons pour lesquelles la réalisation d'un système de mesure de pression d'interface entre un système de compression et la peau, permettant une utilisation simple et routinière, est restée jusqu'à ce jour un problème non résolu.

Le document US 2006/0036203 décrit un dispositif destiné à appliquer une pression contrôlée et modulable sur un membre. Ce dispositif comprend un manchon tubulaire entourant le membre, une pluralité de ballonnets gonflables, et des moyens permettant de gonfler de manière différenciée chacun de ces ballonnets. Il est indiqué que les moyens de gonflage de ces ballonnets sont asservis en fonction d'une comparaison opérée entre les signaux délivrés par des capteurs de pression et des valeurs de consigne correspondantes fonction d'un profil de pression recherché. Ces capteurs sont reliés entre eux par des fils réunis en faisceau courant à l'intérieur du manchon tubulaire. Dans ce document, ces capteurs sont définis de façon très succincte.

Le document WO 2008/102308 décrit un vêtement comportant au moins deux capteurs destinés à mesurer la pression et le couple pression - force de cisaillement afin d'éviter l'apparition d'escarres chez un patient. Ces capteurs sont destinés à mesurer les pressions exercées par le poids du patient ou de ses membres et non pas d'une pression d'interface appliquée par un dispositif de compression.

Dans ces conditions, la présente invention a pour but de résoudre le problème technique consistant en la fourniture d'un système de mesure de pression, utilisable notamment dans le cadre d'un traitement par thérapie compressive, qui permette de mesurer durant plusieurs jours avec précision de faibles pressions, à la fois en mode statique (patient immobile) et en mode dynamique (patient en mouvement) et qui soit capable de fournir un signal reproductible, linéaire, facilement exploitable par un système électronique et permettant de contrôler l'efficacité du traitement.

Il a été découvert, et ceci constitue le fondement de la présente invention, qu'il était possible de résoudre de manière satisfaisante et particulièrement simple ce problème technique en utilisant au moins deux capteurs de pression destinés à mesurer simultanément la pression s'exerçant en deux emplacements prédéterminés du patient à traiter et dont le matériau formant la partie active venant au contact de la peau dans l'application préférentielle envisagée présente un module élastique de compression compris dans une plage de valeurs sélectionnée.

Ainsi, selon un premier aspect, la présente invention concerne un système de mesure d'une pression d'interface exercée sur la peau selon la revendication 1.

Selon une caractéristique particulière de l'invention, le matériau constituant ladite couche intermédiaire précitée est choisi parmi :
- les matériaux alvéolaires, tels qu'en particulier des mousses, de préférence hydrophobes, comme par exemple des mousses en polyéthylène réticulé, en polyuréthane, en silicone, en polychlorure de vinyle, en polyéthylène-propylène diène, en néoprène ;
- les gels, de préférence hydrophobes, comme par exemple des gels de silicone ou de polyuréthane ;
- les mélanges à base de polymères thermoplastiques de préférence hydrophobes, comme par exemple des mélanges de polymères tri-blocs et de plastifiant.

Selon une autre caractéristique particulière de l'invention, chaque capteur de pression est sensiblement plat et choisi parmi les capteurs résistifs, les capteurs piézo-électriques, les capteurs magnétiques à effet Hall, les capteurs capacitifs.

Selon une autre caractéristique particulière de l'invention, le système électronique précité comprend :
- un dispositif embarqué relié au support, de préférence en y étant fixé, et permettant l'acquisition et le traitement des valeurs mesurées par les capteurs de pression, ledit dispositif comportant des moyens de transmission desdites valeurs mesurées par les capteurs ;
- un dispositif déporté comportant :
   - des moyens de communication compatibles avec les moyens de transmission dudit dispositif embarqué et assurant au moins la transmission des données du dispositif embarqué vers le dispositif déporté ; et
   - un circuit de traitement relié à un dispositif d'affichage.

Les systèmes de compression actuellement commercialisés souffrent également de l'inconvénient de ne pas être adaptables à la morphologie du patient (taille de la jambe, dimension du mollet) et à l'évolution de cette morphologie au cours du temps, comme par exemple en cas d'œdème.

Dans ce contexte, la présente invention a également pour but de résoudre le problème technique consistant en la fourniture d'un système de mesure de pression adaptable à la morphologie du patient et à l'évolution de sa pathologie.

Pour résoudre ce deuxième problème technique, et selon une première variante préférée, le support du système de mesure de pression d'interface selon l'invention est extensible.

La conception d'un système de mesure extensible avec une électronique incorporée pose elle aussi de multiples problèmes à résoudre.

La première difficulté réside dans la mise au point d'un système dont les fonctionnalités du circuit électronique et les pistes conductrices reliant les différents éléments fonctionnels (capteurs, microprocesseur, pile, etc.) ne sont pas altérées lors de l'extension.

De la même façon, ces éléments fonctionnels ne doivent pas se désolidariser du support lors de l'extension de ce dernier.

Enfin, le circuit électronique doit être éventuellement protégé, pour être, le cas échéant, partiellement ou totalement réutilisable.

Ces difficultés ont été résolues par différentes caractéristiques additionnelles de l'invention qui seront détaillées par la suite.

L'invention vise également à résoudre un troisième problème technique.

En effet, dans certains cas, en particulier dans le cas d'un traitement d'une plaie chronique de type ulcère, l'utilisation d'un système de compression se heurte au problème additionnel de la surveillance de zone à traiter, en l'occurrence la plaie. Cette dernière est en effet recouverte par un pansement absorbant lui-même caché par le bandage du système de compression.

Si, au cours du traitement, le niveau d'exsudation de la plaie devient supérieur à la capacité d'absorption du pansement, les exsudats débordent de la plaie. Ceci peut conduire, si ce phénomène n'est pas rapidement détecté, à la macération de la plaie et à l'altération de la peau périlésionnelle, entraînant un ralentissement ou une altération du processus de cicatrisation de l'ulcère. De plus, le contact du bandage avec les exsudats peut détériorer les propriétés mécaniques de ce dernier, et par conséquent son efficacité. De plus encore, l'apparition de souillures au niveau du bandage peut être à l'origine de craintes et de problèmes psychologiques pour le patient qui peuvent le conduire à refuser d'utiliser à nouveau un bandage.

Par ailleurs, lors des périodes très chaudes, on assiste à un gonflement des jambes qui modifie la pression appliquée par le système et le patient a très souvent tendance à retirer le bandage.

La mesure de l'humidité et/ou de la température sous le bandage, parallèlement à la mesure de la pression, permettrait donc d'améliorer l'efficacité globale du traitement et d'adapter la pose du bandage à l'évolution de l'environnement de la zone à traiter.

Ainsi, pour résoudre ce troisième problème technique et selon une deuxième variante préférée de l'invention, le support porte en outre :
- au moins un capteur d'humidité et/ou
- au moins un capteur de température.

Selon un second aspect, la présente invention concerne un kit de contention ou de compression qui est constitué d'un système de contention ou de compression et d'un système de mesure de pression d'interface tel que défini précédemment.

### DESCRIPTION DETAILLE DE L'INVENTION

L'invention sera mieux comprise à la lecture de la description explicative qui va suivre faite en référence aux dessins annexés dans lesquels :
- la figure 1 illustre schématiquement un système de mesure de pression d'interface selon un mode de réalisation de l'invention mis en place sur une jambe et montre les points B1 et C au niveau desquels le différentiel de pression est mesuré ;
- la figure 1A est une figure semblable à la figure 1 illustrant un système de mesure de pression d'interface selon un autre mode de réalisation de l'invention mis en place sur une jambe ;
- la figure 2 illustre un support équipé de pistes conductrices selon un mode de réalisation de l'invention ;
- la figure 3 illustre schématiquement l'architecture du système électronique du système de mesure de pression d'interface selon l'invention ;
- les figures 4A à 4C illustrent différents modes de réalisation d'antennes susceptibles d'être utilisées dans le système électronique précité ;
- les figures 5A à 5D illustrent différents modes de réalisation d'un capteur ou système de détection d'humidité susceptible d'être utilisé dans le cadre de l'invention ;
- la figure 6 illustre un mode de réalisation d'un système de mesure de pression d'interface comportant un système de détection d'humidité ;
- la figure 7 illustre un autre mode de réalisation du système de mesure de pression d'interface représenté à la figure 6.

### Le support

Dans le cadre de la présente invention, le support du système de mesure de pression peut être constitué de tout matériau souple, apte à être conformé en plaque et déformable pour épouser la forme de la partie du corps à traiter, par exemple une jambe.

Un tel matériau peut être, par exemple :
- un matériau textile comme un tissé, un non tissé, un tricot ou un textile 3D ;
- un matériau alvéolaire comme une mousse ;
- un film ;
- un complexe associant deux de ces matériaux ;
- une matrice de polymère tel qu'un gel de silicone.

A titre d'exemples de matériaux complexes, on peut citer des complexes constitués de l'association d'un non tissé et d'un film. De tels matériaux peuvent être réalisés par des techniques bien connues de l'homme de l'art comme l'adhésivation, le calandrage à chaud ou le complexage par ultrasons.

Dans le cadre de la thérapie compressive et en particulier des ulcères de jambe, il est important que le système de mesure de pression d'interface n'altère pas la peau du patient qui est souvent fragilisée ou en très mauvais état, en particulier autour de l'ulcère.

Le support devra donc être fin, souple et surtout non occlusif c'est-à-dire qu'il devra présenter une perméabilité à la vapeur d'eau, mesurée selon la norme EN-13726, supérieure ou égale à 1000 g/m²/24 h.

Dans le cadre de la présente invention, des exemples préférés de supports sont les non tissés et les films en polyuréthane.

Selon un mode de réalisation particulièrement préféré de l'invention, le support est extensible, de préférence de façon anisotrope.

A cet effet, parmi les matériaux décrits précédemment, on choisira préférentiellement ceux qui présentent une extensibilité de l'ordre de 10 à 20 % dans le sens longitudinal et une extensibilité de l'ordre de 3 à 12 % dans le sens transversal. Il a été observé qu'une extensibilité de l'ordre de 10 à 20 % dans le sens longitudinal permet de couvrir tous les types de morphologie de patient, à l'aide d'un ou deux produits.

De préférence encore, on utilisera un matériau extensible qui possède un faible module d'élasticité afin de pouvoir concilier les avantages d'extensibilité et l'aptitude du support à adhérer à la peau si ce dernier est adhésivé pour faciliter sa pose.

En effet, plus le matériau extensible présente un module d'élasticité faible et plus sa force de retour est faible. Un support présentant un module d'élasticité relativement faible, par exemple de l'ordre de 40 N/cm ou moins, pourra ainsi rester en extension le temps suffisant pour que le personnel soignant pose aisément le système de compression sur ce dernier.

Selon une variante préférée de l'invention, ce faible module d'élasticité peut être obtenu en formant des trous ou des parties évidées dans le matériau extensible constituant le support. Bien évidemment, ces trous ou parties évidées seront disposés sur le support en des emplacements convenablement choisis pour ne pas altérer, lors de son extension, les connections électroniques et les pistes conductrices éventuellement présentes sur celui-ci.

A titre d'exemples de matériaux extensibles particulièrement préférés, on peut citer les non tissés ou les films à base de polyuréthane, de polyétherpolyester (en particulier les produits commercialisés sous les dénominations HYTREL® ou ARNUEL®), de polyamide ou de polyéther-polyamide.

### Les capteurs de pression

Le système de mesure de pression d'interface selon l'invention comprend un ensemble de capteurs, portés par le support décrit précédemment, dont au moins deux capteurs de pression disposés de façon espacée sur le support pour permettre de mesurer la pression s'exerçant en deux emplacements dont l'un est prédéterminé.

La thérapie compressive s'applique dans la plupart des cas sur les membres inférieurs.

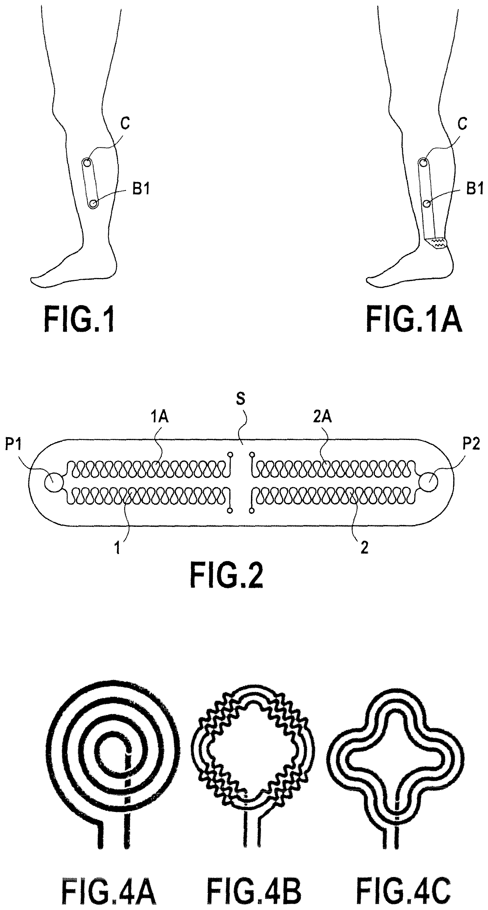

A titre d'exemple, on a donc représenté à la figure 1, un système destiné à être placé sur la jambe.

Dans ce cas, les deux capteurs de pression précités seront avantageusement fixés sur le support, en des emplacements choisis de telle sorte que ces capteurs soient respectivement disposés, en position d'utilisation du système de mesure :
- d'une part, au niveau d'un emplacement prédéterminé tel que la zone référencée B1, où le tendon d'Achille se transforme en muscle du mollet (10 à 15 cm environ au dessus de la malléole) ; et
- d'autre part, au niveau d'une zone quelconque située en « aval » de la zone B1, (c'est-à-dire au dessus de la zone B1 en remontant le long de la jambe) de préférence au niveau de la zone référencée C où le mollet atteint son périmètre maximum.

L'efficacité d'un système de compression dépend en effet du maintien d'un gradient de pression entre ces deux zones, la pression au niveau de la zone B1 devant être supérieure à la pression au niveau d'une zone située en aval de cette dernière pour garantir que le sang soit chassé vers le haut et ne stagne pas. De ce fait, il est extrêmement important de pouvoir mesurer la pression au niveau de ces deux zones.

Chaque capteur de pression répond avantageusement à des critères géométriques (surface, épaisseur) et des exigences métrologiques (sensibilité et précision de l'ordre de 1 mm de mercure, reproductibilité des mesures pendant plusieurs jours) spécifiques.

De préférence, chaque capteur doit aussi être capable de subir une surcharge de 100 % sans détérioration, c'est-à-dire une pression de 250 mm de mercure.

Pour répondre à ces contraintes, on choisira de préférence un capteur de pression dont la partie active (transducteur associé à une couche intermédiaire le recouvrant) :
- est sensiblement plate
- présente une épaisseur inférieure ou égale à 3 mm sous contrainte de 20 mm de mercure ;
- présente une surface comprise entre 0,25 et 3 cm² ;
- permet de mesurer avec précision une pression d'interface dans une plage variant de 1 à 120 mm de mercure et en particulier de 5 à 60 mm de mercure.

Ainsi, un capteur de pression optimale présente une linéarité supérieure à 0,95, et de préférence supérieure ou égale à 0,98, sur la zone de 1 à 120 mm de mercure et en particulier de 5 à 60 mm de mercure.

Pour assurer des mesures fiables et reproductibles dans le temps, les inventeurs ont en outre déterminé, et ceci constitue une caractéristique essentielle de l'invention, que le matériau constituant la couche intermédiaire recouvrant le transducteur doit présenter un module élastique de compression mesuré au niveau de la surface destinée à venir au contact de la peau compris entre 30 et 500 kPa.

Ce module élastique de compression peut être mesuré à l'aide d'un dynamomètre équipé de plateaux de compression suivant la procédure décrite dans la norme ISO 844 de 2007.

Le matériau constituant la couche intermédiaire recouvrant le transducteur présente un module élastique de compression compris entre 80 et 400 kPa et de préférence encore compris entre 200 et 400 kPa.

Tout type de capteur de pression, à l'exception des capteurs pneumatiques, pourra être utilisé dans le cadre de l'invention.

On peut ainsi citer des capteurs résistifs, des capteurs piézo-électriques ou capacitifs dont les propriétés électriques varient en fonction de la pression exercée ou des capteurs magnétiques qui fonctionnent sous le principe de l'effet « Hall ».

D'une façon générale, de tels capteurs comprennent :
- d'une part, une partie active constituée d'un élément transducteur apte à convertir la pression en un signal, et
- d'autre part, une partie non active comportant des contacts ou des pistes conductrices aptes à transmettre le signal émis par le transducteur à une unité de traitement.

Dans le cadre de la présente demande le terme « capteur de pression » désigne donc l'association d'un transducteur et des contacts ou pistes conductrices précitées.

Dans les capteurs de pression utilisés dans le cadre de l'invention, le transducteur traduit directement la pression qui lui est appliquée en un signal, par exemple électrique ou magnétique, traduisant une variation d'une des caractéristiques physiques intrinsèques de l'élément transducteur comme en particulier sa résistance, sa capacité, son champ électrique ou encore son effet QTC (Quantum Tunnelling Effect).

Dans ces capteurs, l'élément transducteur ne comprend ni gaz, ni liquide, à la différence des capteurs pneumatiques.

Il est à noter que l'utilisation de tels capteurs pneumatiques ne peut être envisagée dans le cadre de la présente invention :
- d'une part, dans la mesure où de tels capteurs sont généralement couteux, encombrants et complexes ; et
- d'autre part dans la mesure où leur fiabilité est rendue incertaine en raison des possibles variations, par exemple sous l'effet de la température, des propriétés du gaz ou du liquide qu'ils contiennent et des risques de fuite en cas de rupture de la membrane enveloppant ce gaz ou ce liquide.

Parmi les capteurs résistifs utilisables dans le cadre de la présente invention, on citera les capteurs commercialisés par la société TEKSCAN sous la dénomination FLEXIFORCE® ainsi que ceux commercialisés par la société INTERNATIONAL ELECTRONICS AND ENGINEERING IEE sous la dénomination FSR®. On préférera plus particulièrement les capteurs commercialisés par la société IEE sous la dénomination FSR®.

Quel que soit le type de capteur utilisé parmi ceux mentionnés précédemment, la surface et l'épaisseur de ces capteurs devront être modifiées par l'ajout d'une couche intermédiaire pour optimiser les contacts entre le transducteur, la peau et le système de compression afin d'éviter l'effet « Hamac » et de garantir une mesure linéaire et reproductible dans le temps.

Les capteurs commercialisés par la société IEE sont des résistances de détection de force (Force Sensicy Resistor en anglais). Leur structure est extrêmement simple. Il s'agit d'une structure multicouches formée de deux feuilles de polymère laminées ensemble qui enveloppent deux électrodes et une résistance. Plus la pression appliquée sur cette structure est élevée, plus leur résistance décroit. Ces capteurs résistent à la température, aux produits chimiques et à l'humidité. Les technologies et la mise en œuvre de tels capteurs sont par exemple décrites dans les demandes de brevet WO 2006/79581 et WO 2006/58880 de la société IEE.

Pour optimiser le fonctionnement de ces capteurs connus et obtenir le module élastique de compression idéal, la face du transducteur venant au contact avec la peau doit être recouverte d'une couche plus ou moins épaisse d'un matériau qui présente un module élastique de compression compris entre 30 et 450 kPa.

En effet, ces capteurs connus sont plats, rigides, et de très faible épaisseur, de sorte qu'ils ne peuvent pas être directement utilisés dans l'application envisagée.

Si nécessaire, les deux faces du transducteur pourront être recouvertes avec des matériaux identiques ou différents, en particulier des matériaux présentant un module élastique de compression comprise entre 30 et 500 kPa. Ceci permet de diminuer plus significativement l'effet négatif de la pression appliquée par le système de compression sur la surface du capteur opposée à la peau et d'obtenir ainsi une meilleure sensibilité et linéarité du capteur.

Toutefois dans le cadre de la présente invention, on évitera d'utiliser un capteur trop épais, au niveau de sa partie active (transducteur recouvert d'une couche intermédiaire) par exemple dont l'épaisseur est supérieure à 5 mm, et qui pourrait rendre difficile sa fixation sur le support et agresser la peau du patient qui est souvent fragilisée.

En d'autres termes, la structure finale du capteur, et en particulier l'épaisseur de sa partie active, sera choisie en recherchant le meilleur compromis entre sa bonne compressibilité et sa sensibilité. Ainsi, si l'on cherche à mesurer des pressions et des variations de pressions faibles, on privilégiera un capteur dont seule la face du transducteur venant en contact avec la peau est recouverte d'un matériau présentant un module élastique de compression compris entre 30 et 500 kPa et en particulier compris entre 200 et 400 kPa.

Parmi les matériaux utilisables pour obtenir un tel module élastique de compression, on citera les matériaux alvéolaires, comme par exemple les mousses en polyéthylène réticulé physiquement, et en particulier les mousses de la gamme ALVEOLIT commercialisées par la société ALVEO ou celles commercialisées par la société TROCELEN sous différentes références. Ces mousses présentent une excellente résistance chimique et sont inertes à l'eau.

On peut encore citer des mousses en polyuréthane, en silicone (par exemple les mousses silicone commercialisées par la société PORON sous les références UL94HF ou UL94VO), des mousses en PVC (chlorure de polyvinyle), en EPDM (éthylène-propylène diène monomère) ou en néoprène.

Toutes ces mousses peuvent être à cellules ouvertes ou fermées.

Dans le cadre de la présente invention, on utilisera de préférence une mousse hydrophobe afin d'éviter que la transpiration ou les exsudats de la plaie ne viennent modifier leurs propriétés d'élasticité, de densité, leur module de compression voire leur épaisseur, et de préférence encore une mousse hydrophobe sans plastifiant afin d'éviter l'éventuelle migration de produits toxiques sur la peau ou dans la plaie.

D'une façon générale, on utilisera des mousses présentant une épaisseur comprise entre 0,3 et 5 mm, de préférence entre 0,5 et 1,5 mm et une densité comprise entre 25 et 250 kg/m³, que l'on associera de préférence à des transducteurs présentant une épaisseur inférieure à 0,8 mm, de préférence inférieure à 0,5 mm, et, de préférence encore inférieure à 0,3 mm.

Selon la variante de réalisation actuellement préférée de la présente invention, chaque capteur de pression est un capteur FSR® comprenant un transducteur ayant une épaisseur de 0,4 mm, une surface venant en contact avec la peau de 2,27 cm² et portant une mousse de polyuréthane (commercialisée par la société PORON).

Selon d'autres variantes de réalisation de l'invention ce même capteur FSR® a été recouvert des couches intermédiaires suivantes :
a) Couche à base d'un mélange de polymères thermoplastiques et de plastifiant.
   Ce mélange était constitué de :
   - 88 % en poids de copolymère tribloc commercialisé par la société Kraton sous la dénomination Kraton® G1651 ; et
   - 12 % en poids d'huile minérale commercialisée par la société Shell sous la dénomination Ondina® 917.

   Ce mélange a été déposé sous forme d'une couche présentant une épaisseur est de 4,24 mm sur le capteur FSR® précité.
   Le module élastique de cette couche était de 240 kPa.
b) Couche à base d'une mousse de polyuréthane commercialisée par la société PORON sous la référence 4701-50-30031-04 de couleur noire.
   Sur un capteur FSR® précité, on a fixé à l'aide d'un adhésif double face de 90 µm d'épaisseur (adhésif acrylique/ polyéthylènetéréphtalate/adhésif acrylique) une couche de 0,8 mm d'épaisseur de la mousse précitée.
   Le module élastique de compression de cette couche de mousse était de 380 kPa .
c) Couche à base de mousse de polyuréthane commercialisée par la société PORON de couleur bleue.
   Sur un capteur FSR® précité, on a fixé à l'aide d'un adhésif double face de 90 µm d'épaisseur (adhésif acrylique/ polyéthylènetéréphtalate/adhésif acrylique) une couche de 1,45 mm d'épaisseur de la mousse précitée.
   Le module élastique de compression de cette couche de mousse était de 380 kPa.
d) Sur chacune des deux faces d'un capteur FSR® précité, on a fixé à l'aide d'un adhésif double face de 90 µm d'épaisseur (adhésif acrylique/ polyéthylènetéréphtalate/adhésif acrylique) une couche de 1,45 mm d'épaisseur des mousses décrites en b) et c) ci-dessus.

Selon d'autres variantes de réalisation de l'invention, on a remplacé dans les variantes décrites précédemment le capteur FSR® par un capteur FlexiForce de référence 1-617-464-4500, commercialisé par la société Tekscan.

Dans tous les cas, les capteurs ainsi modifiés se sont avérés satisfaisants pour atteindre les résultats recherchés.

Ces matériaux alvéolaires seront fixés sur le transducteur du capteur selon des techniques connues, par exemple par calendrage à chaud, par ultrasons si les composants sont thermofusibles ou par adhésivation.

De préférence, cette fixation sera réalisée par adhésivation à l'aide d'un adhésif double-face, par exemple à base d'acrylique, présentant de préférence une épaisseur inférieure à 200 µm, de préférence encore inférieure à 100 µm.

Comme autres matériaux permettant d'ajuster le module élastique de compression de la surface active du capteur venant en contact avec la peau, on peut aussi citer les gels, et en particulier les gels de silicone ou de polyuréthane. Comme dans le cas des mousses, on préférera utiliser des gels hydrophobes afin que leurs propriétés ne soient pas altérées par la transpiration ou les exsudats.

On peut encore citer des mélanges de polymères thermoplastiques et de plastifiant. Ces polymères thermoplastiques seront notamment des polymères triblocs, comme en particulier les polymères triblocs acryliques référencés par la société KURARAY sous le terme "LA polymers", les polymères triblocs du type ABA dans lesquels A représente des unités styrène et B des unités butadiène, isoprène, éthylène-butylène ou éthylène propylène, tels que les produits des gammes KRATON G et KRATON D commercialisés par la société KRATON, ces derniers pouvant être associés à une huile minérale.

De tels mélanges de polymères thermoplastiques et de plastifiant sont bien connus de l'homme de l'art.

Ces matériaux pourront être assemblés avec le transducteur selon les techniques précédemment citées et en particulier par adhésion. Afin de faciliter le collage et de garantir sa solidité, on utilisera de préférence dans ce cas un adhésif qui aura une nature chimique identique à celle du matériau à fixer, comme par exemple un adhésif silicone, un adhésif polyuréthane ou un adhésif hot melt à base de copolymère tribloc acrylique ou ABA pour assembler respectivement un gel de silicone, un gel de polyuréthane ou un matériau à base de copolymères triblocs et de plastifiant.

Selon une variante de réalisation, dans le cas où l'on désire appliquer un gel ou un matériau à base de copolymère tribloc et de plastifiant sur les deux faces du transducteur du capteur, ce dernier pourra être enrobé, par exemple par moulage, dans ledit gel ou ledit matériau.

Enfin, pour faciliter le contact du capteur sur la peau, on peut envisager d'appliquer un gel adhésif directement sur le transducteur. Un tel gel peut être notamment un gel de silicone ou un adhésif à base d'un mélange de copolymère tribloc, de plastifiant et de résine tackifiante.

Selon une autre variante de réalisation qui n'est pas dans le cadre de l'invention revendiquée, le capteur peut être utilisé tel quel s'il présente un module élastique de compression adéquate sans ajout d'un quelconque matériau supplémentaire, sur la face du transducteur venant en contact avec la peau.

A titre d'exemple de tels capteurs, on peut citer un capteur à effet Hall dont le transducteur se présente sous la forme d'un bloc monocouche de matériau alvéolaire, par exemple une mousse d'éthylène et d'acétate de vinyle, chargé de particules magnétiques permettant de transformer la pression appliquée en une valeur électrique mesurable.

Dans le cas d'un tel capteur comportant un transducteur monocouche, on privilégiera l'utilisation d'un capteur dont le transducteur présente une épaisseur comprise entre 0,5 et 4,5 mm, de préférence entre 1 et 3mm.

### Le système électronique

Le système de mesure de pression d'interface selon l'invention comprend également un système électronique relié aux capteurs de pression et apte à acquérir les valeurs mesurées simultanément par ces capteurs.

C'est l'acquisition de ces valeurs qui permet notamment de contrôler à tout moment le différentiel de pression exercée par le système de compression et par conséquent l'efficacité de celui-ci.

Dans le cadre de la présente invention, on entend par « système électronique » l'ensemble des éléments utilisés pour permettre les échanges de flux de données et d'énergie nécessaires pour gérer les mesures réalisées par les capteurs de pression.

D'une façon générale, ce système électronique comprend :
- au moins une unité de traitement comprenant un micro contrôleur et une mémoire, ladite unité de traitement étant apte à acquérir et à traiter les signaux représentatifs des valeurs mesurées par les capteurs de pression ;
- au moins un circuit d'alimentation comprenant une source d'énergie et un dispositif convertisseur et distributeur d'énergie.

Selon un mode de réalisation actuellement préféré de l'invention, ce système électronique comprend :
- un dispositif embarqué relié au support, de préférence en y étant fixé, et permettant l'acquisition et le traitement des valeurs mesurées par les capteurs de pression, ledit dispositif comportant des moyens de transmission desdites valeurs mesurées par les capteurs ;
- un dispositif déporté comportant :
   - des moyens de communication compatibles avec les moyens de transmission dudit dispositif embarqué et assurant au moins la transmission des données du dispositif embarqué vers le dispositif déporté ; et
   - un circuit de traitement relié à un dispositif d'affichage.

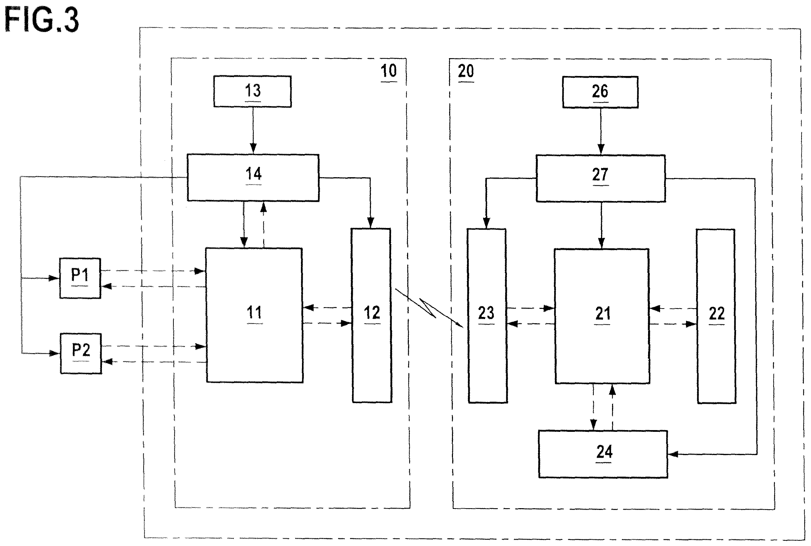

L'architecture générale de ce système électronique préféré est illustrée sur la figure 3.

Le dispositif embarqué 10 comprend une unité de traitement 11 comprenant un micro contrôleur et des moyens de transmission 12 desdites valeurs mesurées par les capteurs de pression P₁ et P₂ ainsi qu'un circuit d'alimentation comprenant une source d'énergie 13 et un dispositif convertisseur et distributeur d'énergie 14.

Le micro contrôleur de l'unité de traitement 11 intègre, filtre, traite et numérise les signaux bruts émis par les capteurs de pression P₁ et P₂. Il effectue la conversion analogique/numérique de ces signaux. Il peut également analyser ces signaux par transformée de Fourrier ou utiliser des filtres numériques combinés à un logiciel pour vérifier des données sur certains événements. Il stocke les données d'étalonnage et de calibration des capteurs de pression et les données mesurées par ces capteurs. Il contrôle la transmission des données vers le dispositif déporté et la bonne gestion de la distribution d'énergie via le dispositif convertisseur et distributeur d'énergie 14.

Dans le cadre de la présente invention, on pourra utiliser tout type de micro contrôleur (ou microprocesseur) couramment employé dans la réalisation de systèmes électroniques et microélectroniques. De tels produits sont par exemple commercialisés par les sociétés TEXAS INSTRUMENT, MICROCHIP ou NXP respectivement sous les dénominations 8150, 16F690, Coolflux.

Le dispositif déporté 20 comprend une unité de traitement 21 comprenant un micro contrôleur, une mémoire 22, des moyens de communication 23 compatibles avec les moyens de transmission 12 du système embarqué, un dispositif d'affichage 24 et un circuit d'alimentation comprenant une source d'énergie 26 et un dispositif convertisseur et distributeur d'énergie 27.

La communication entre le système embarqué et le système déporté peut être filaire ou sans fil (module radio et antenne) comme dans l'exemple représenté.

### La source d'énergie

Dans le cadre de la présente description, on entend par "source d'énergie" tout dispositif apte à fournir l'énergie suffisante pour alimenter tous les éléments du système de mesure de pression d'interface (capteurs et système électronique).

Dans le cas d'un système filaire, cette énergie pourra être fournie par exemple à partir d'une prise électrique via un fil.

Dans le cas d'un système sans fil, on pourra utiliser tout type de source d'énergie couramment employé avec des appareils de mesure, comme en particulier des piles jetables, des batteries rechargeables ou des dispositifs capables de récupérer l'énergie de leur environnement désignés sous le terme anglais de « scavenger ». Un « scavenger » peut notamment recueillir l'énergie solaire ou l'énergie produite par des gradients de température, des vibrations, des mouvements, notamment par le déplacement d'une personne, ou des phénomènes électromagnétiques. Un « scavenger » convertit l'énergie ainsi recueillie en données électriques (tension, fréquence...). Le stockage de cette énergie peut être effectué dans une batterie ou un super condensateur pouvant être incorporé dans le système de mesure de pression d'interface.

### Les moyens de communication

Selon un mode de réalisation actuellement préféré de l'invention, la communication entre le dispositif embarqué et le dispositif déporté se fera sans fil et de préférence par radiofréquence.

Les moyens de communication comprennent généralement un module de communication qui incorpore l'électronique permettant de réaliser les communications et une antenne avec le software et le hardware adaptés.

Le module de communication du dispositif embarqué peut être :
- unidirectionnel et assurer la seule transmission vers l'extérieur des données fournies par les capteurs et le microcontrôleur ; ou
- bidirectionnel (Emetteur/Récepteur) et dans ce cas recevoir en plus des données venant de l'extérieur, comme dans l'exemple représenté.

La communication sans fil pourra éventuellement se faire en deux étapes.

Dans une première étape on réalise la communication des données entre le système embarqué et un boîtier de contrôle portable. Ce dernier pourra par exemple se présenter sous la forme d'un bracelet-montre ou d'un dispositif type téléphone portable. Dans ce cas, la portance entre le système et le boîtier sera de préférence de l'ordre de dix mètres et le débit de données maximum inférieur à un kilobit par seconde. La communication sera avantageusement réalisée par radiofréquence, (par exemple une radiofréquence de 2,4 GHertz) et on pourra notamment utiliser à cet effet le module de communication commercialisé par la société NORDIC SEMICONDUCTEUR sous la référence nRF24L01. Alternativement, la communication peut être également réalisée par couplage inductif, avec l'avantage corrélatif d'un très faible niveau d'interférence avec le corps humain, et on pourra utiliser à cet effet le module de communication commercialisé par la société NXP sous la référence "Coolflux radio module".

Dans une deuxième étape, on réalise la communication entre ce boîtier de contrôle portable et un ordinateur. On préférera dans ce cas une liaison radiofréquence de 2,45 GHertz qui permet des transferts de données plus importants et à des distances plus éloignées. Dans ce cas, le débit de données est de l'ordre de un Mégabit par seconde et la portance de l'ordre de cent mètres. Divers dispositifs peuvent être utilisés à cet effet et l'on citera par exemple les protocoles Zigbee®, bluetooth® et Wifi®.

Une communication en deux étapes est particulièrement adaptée pour une utilisation du système de mesure de pression d'interface selon l'invention par le personnel soignant voire par le patient lui-même.

Le boîtier de contrôle portable permet de contrôler le système et d'enregistrer des données. Ces données peuvent être exploitées par la suite par le médecin sur son ordinateur pour adapter le traitement et vérifier l'observance du patient.

Bien entendu, une communication directe des données entre le système embarqué et un ordinateur est également envisageable.

Lorsque la communication utilise la radiofréquence, le dispositif embarqué comprend une antenne. Celle-ci peut avoir différentes configurations selon la fréquence utilisée, la forme du signal à traiter et l'énergie disponible.

D'une façon générale, on utilisera une antenne en cuivre ou en aluminium mais on peut également utiliser une antenne formée d'une encre conductrice.

Selon un mode de réalisation préféré, l'antenne se présente sous la forme d'une bobine cylindrique enroulée autour d'un cœur en Ferrite (de diamètre inférieur ou égal à 4 mm), ou d'une bobine plate en forme d'escargot (de diamètre supérieur ou égal à 10 mm).

Comme les autres composants du dispositif embarqué, l'antenne peut être fixée sur un circuit imprimé rigide ou flexible avec ces autres composants ou de façon isolée sur le support. De même, elle pourra être enrobée de façon isolée ou avec ces autres composants.

Dans le cas où le support est extensible, on préférera fixer l'antenne directement sur ce support ou sur un circuit extensible tel qu'il sera décrit plus loin, comme par exemple un film polyuréthane extensible.

Les figures 4A, 4B et 4C illustrent diverses formes d'antennes qui peuvent être déposées directement sur le support extensible.

### Les pistes conductrices

Selon un mode de réalisation actuellement préféré, le dispositif embarqué sera relié aux capteurs de pression par des pistes conductrices.

Dans le cas où le support n'est pas extensible, les pistes conductrices pourront être réalisées par des techniques bien connues de l'homme du métier comme en particulier par sérigraphie à l'aide d'une encre conductrice, par exemple à base d'argent ou d'un mélange argent-cuivre.

Ces techniques peuvent être également utilisées dans le cas où ledit support est extensible.

Les pistes conductrices peuvent être également intégrées à l'intérieur du support lorsque celui-ci se présente sous la forme d'une plaque de polymère extensible, en particulier d'un gel de silicone ou de polyuréthane.

Alternativement, des pistes conductrices, par exemple en cuivre, peuvent être formées sur le support lorsque celui-ci se présente sous la forme d'un film, par exemple de polyuréthane. En pratique, ceci peut être réalisé en recouvrant une plaque de cuivre sur une de ses faces par un film de polyuréthane, en appliquant un masque sur la face non recouverte de la plaque (les parties pleines dudit masque présentant la forme des pistes à obtenir) et en détruisant le cuivre non masqué par attaque acide.

Lorsque le support est extensible, il est important qu'il n'y ait pas de rupture de ces pistes conductrices lors de l'extension du support. A cet effet, les pistes se présenteront de préférence sous la forme d'une succession d'éléments en forme d'Oméga reliés deux à deux successivement. Lors de l'extension du support, chaque élément en forme d'Oméga peut se déformer longitudinalement en évitant ainsi la rupture de la piste. De telles structures en Oméga sont par exemple décrites dans la demande de brevet WO 2004/107973.

Alternativement, il est possible d'éviter les risques de rupture en fixant des dispositifs limiteurs d'extension sur le support extensible.

Ces dispositifs limiteurs d'extension peuvent être par exemple constitués de barrettes de rigidification. Ces barrettes seront formées d'un matériau présentant une extensibilité plus faible que celle du support extensible. Ainsi lorsque le support est étiré, ce dernier se trouvera "bloqué" par lesdites barrettes à partir d'une certaine extension. De préférence, ces barrettes présenteront une extensibilité longitudinale de l'ordre de 20 % ou moins. Les matériaux susceptibles d'être utilisés pour réaliser ces barrettes pourront être similaires à ceux cités pour le support, en particulier des textiles, des mousses ou des films. Ces barrettes pourront être fixées sur le support extensible par des technologies classiques adaptées à la nature du support. A titre d'exemples de telles les technologies, on peut citer le faufilage, l'aiguilletage pour un support textile, comme par exemple un non tissé, ou pour une mousse; la soudure par ultrasons, le collage, le complexage thermique ou la fixation à l'aide d'un adhésif pour un support en film.

Les dispositifs limiteurs d'extension peuvent également être constitués d'un ou plusieurs fil(s) intégré(s) dans le plan du support, sur tout ou partie de celui-ci selon une configuration en forme d'onde sinusoïdale, et présentant une résistance à la traction supérieure à celle du support. Ainsi, lorsque le support est étiré, le fil se tend et bloque l'extension du support. Ce ou ces fils pourront être intégrés ou fixés sur le support extensible selon les techniques précédemment décrites pour la fixation des barrettes.

D'une façon générale, on positionnera l'ensemble des composants du dispositif embarqué entre les deux capteurs de pression afin de diminuer les risques de rupture des pistes conductrices mises sur le support. Plus ces pistes seront courtes moins leur risque de rupture sera élevé.

On a représenté à la figure 2 un mode de réalisation d'un support S comportant deux séries de pistes conductrices 1,1A et 2,2A formés d'éléments en Oméga et destinées à être respectivement reliées aux capteurs de pression P₁ et P₂.

### Les connexions

Les éléments fonctionnels du dispositif embarqué et les capteurs seront connectés aux pistes conductrices du support selon les techniques classiques utilisées dans le cas de supports non extensibles.

Ces connexions peuvent être des soudures classiques ou bien encore réalisées :
- soit à l'aide d'adhésifs conducteurs en Z, comme par exemple les adhésifs commercialisés par la société ADHESIVE RESEARCH sous les dénominations ARCARE 90366 ou ARCARE 90447,
- soit à l'aide d'adhésifs conducteurs sous forme de mousse adhésivée, de colles conductrices ou de polymères conducteurs comme par exemple les produits commercialisés par les sociétés DOW CORNING et EMERSON AND CUMING respectivement sous les dénominations DA 65224 et XCS 80091,
- soit encore à l'aide de Velcro conducteur.

De telles connexions vont relier les nœuds conducteurs (dénommés « pads » en anglais) desdits éléments fonctionnels et des capteurs aux nœuds conducteurs disposés sur les pistes conductrices du support.

Alternativement, ces connexions peuvent être réalisées à l'aide de micro-connecteurs couramment employés pour réaliser des connexions dans les ordinateurs ou les téléphones mobiles.

Lorsque le système de mesure est jetable, des connexions inviolables comme des soudures peuvent être utilisées.

En revanche, lorsque le système de mesure est destiné à être partiellement réutilisable, on utilisera, de préférence, des connexions "réactivables", comme par exemple des connexions réalisées à l'aide d'adhésifs ou de micro-connecteurs. Dans ce cas, le support pourra être jeté après utilisation et les éléments fonctionnels réutilisés avec un nouveau support auquel ils seront fixés au moyen de nouveaux adhésifs ou micro-connecteurs. Alternativement, seuls certains éléments fonctionnels du système pourront être réutilisés, par exemple lorsque l'on souhaite changer les capteurs ou la source d'énergie.

Lorsque le système de mesure est destiné à être entièrement réutilisable, l'ensemble des éléments fonctionnels du système (capteurs et système embarqué) pourra être encapsulé ou enrobé, comme les dispositifs électroniques classiques, dans des polymères de type silicone, polyuréthane ou dans des adhésifs comme décrit par exemple dans la demande de brevet WO 2006/094513. On obtiendra dans ce cas un système nettoyable après utilisation et réutilisable un grand nombre de fois.

Dans le cadre de la présente invention, on préfère n'encapsuler que les éléments les plus sensibles, et en particulier les composants électroniques, afin de ne pas altérer les propriétés de "respirabilité" du système.

De la même façon, pour ne pas restreindre l'extensibilité du support, on préfère encore n'encapsuler que les connexions entre les composants électroniques et le support extensible.

Les différents composants du dispositif embarqué (source d'énergie, antenne, microprocesseur) et les pistes conductrices les reliant, selon leur nature et leur taille, pourront être connectés directement aux « pads » réalisés sur le support extensible ou assemblés sur un ou plusieurs éléments "interposeurs" ou circuits imprimés qui seront connectés aux « pads » du support extensible.

Alternativement certains de ces éléments, comme la source d'énergie ou l'antenne, pourront être connectés directement ou via des éléments interposeurs sur une partie prolongeant le support au delà de la partie s'étendant entre les capteurs de pression afin de ne pas altérer l'extensibilité du support. Cette partie prolongeant le support ne sera pas nécessairement extensible.

Comme indiqué précédemment cette partie prolongeant le support et les éléments qui lui sont connectés pourront être encapsulés dans un matériau adéquat comme un silicone à base de poly-diméthyle siloxane par exemple.

Les matériaux utilisables pour la réalisation des éléments interposeurs sur lesquels seront assemblés les composants électroniques sont ceux qui sont couramment utilisés pour la réalisation de circuits électroniques classiques. Même si on peut utiliser des matériaux rigides, on préférera néanmoins utiliser à cet effet des matériaux flexibles, souples ou semi-rigides conformables afin de s'adapter à la morphologie du patient et au rayon de courbure critique d'une cheville qui est de l'ordre de 25 mm.

Parmi les matériaux utilisables pour la réalisation de ces éléments interposeurs souples ou semi-rigides on peut citer les polyimides comme par exemple les produits commercialisés par la société Dupont sous la dénomination KAPTON® et les films époxy fins ayant une épaisseur de l'ordre de 0,1 à 0,4 mm comme les produits commercialisés sous le terme "époxy FR4".

Si nécessaire, en particulier dans le cadre du traitement des ulcères de jambe, on pourra réaliser un vernissage de la surface de ces éléments interposeurs, pour éviter tout court-circuit ou dysfonctionnement électrique qui pourrait être occasionné par le contact avec les exsudats de la plaie ou l'humidité du pansement sous le bandage lors de l'utilisation du système de mesure de pression d'interface. Des vernis utilisables à cet effet sont par exemple les produits PROPOCURE, par exemple série 520-530 commercialisés par la société APCIS.

### Les autres capteurs

Selon diverses variantes de réalisation , le système de mesure de pression d'interface selon invention peut comprendre d'autres capteurs que des capteurs de mesure de pression afin de mesurer des paramètres complémentaires utiles dans le cadre du traitement de la thérapie compressive, comme l'accélération (pour détecter et évaluer l'activité physique du patient), la température (qui joue un rôle important sur la circulation veineuse) et l'humidité (pour détecter les phénomènes de sudation sous le bandage et les problèmes liés au niveau d'exsudation de la plaie dans le cas des ulcères de jambe).

Bien entendu, d'autres paramètres pourront être évalués comme par exemple le pH à l'aide de capteurs physico-chimiques.

De façon préférentielle, afin de ne pas altérer les propriétés d'extensibilité du système de mesure de pression d'interface, ces capteurs seront positionnés sur une partie prolongeant le support au delà de la partie s'étendant entre les capteurs de pression. Cette partie du support pourra être extensible ou non.

Dans le cadre du traitement des ulcères de jambe la détection du degré d'humidité du pansement qui recouvre la plaie et qui est caché sous le bandage est un élément important pour favoriser l'observance du traitement par le patient et garantir l'efficacité du traitement.

Il existe de nombreux dispositifs connus permettant de détecter l'humidité (ou capteurs d'humidité).

Dans le cadre de la présente invention, on préfère cependant utiliser un système constitué de deux électrodes (une jouant le rôle d'anode et l'autre de cathode) espacées d'une distance de quelques millimètres (de préférence de 2 à 20 millimètres, et de préférence encore de 2 à 5 millimètres). Cet espacement sera adapté en fonction des objectifs recherchés : valeur du seuil de détection, localisation sur le support extensible, dimension du pansement et du support extensible et en fonction de la morphologie du patient.

Ces électrodes pourront être constituées de différents matériaux qui seront déposés sur le support, comme les pistes conductrices. Avantageusement, il s'agira d'électrodes formées de pistes en cuivre dont la surface a fait l'objet ou non d'un traitement chimique ou électrolytique, d'un apport d'or ou de platine, ou bien encore d' électrodes obtenues par sérigraphie avec des encres conductrices, comme des encres chargées en sel d'argent, de carbone, etc.

Dans le cas où le support est formé d'un matériau textile, on pourra également utiliser des fibres électroniques conductrices en tant qu'électrodes.

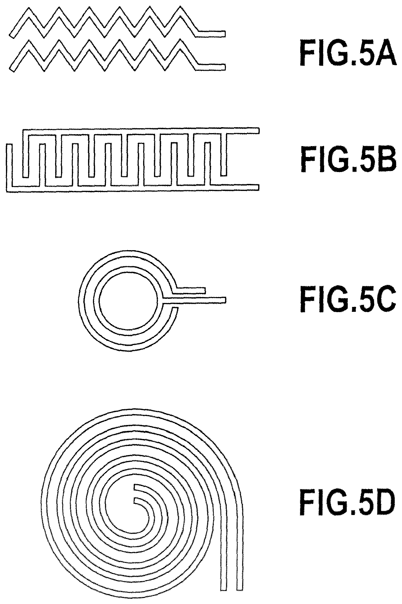

La longueur, la largeur et la configuration de ces électrodes peuvent être variées. On peut ainsi imaginer deux électrodes sinusoïdales parallèles, deux peignes imbriqués l'un dans l'autre, deux anneaux concentriques ou même deux spirales parallèles. De telles configurations sont respectivement représentées sur les figures 5A, 5B, 5C et 5D.

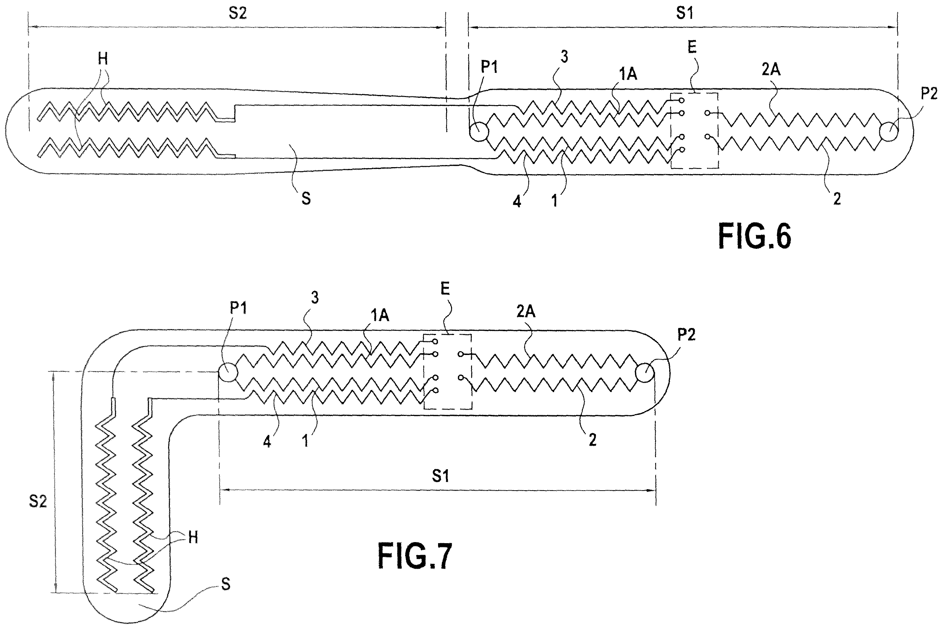

Ces deux électrodes peuvent être reliées au système électronique via des pistes conductrices 3, 4 comme représenté sur la figure 6.

Le principe de la mesure d'un tel détecteur d'humidité est basé sur l'évolution de la valeur de la résistivité entre l'anode et la cathode lors de l'hydratation.

Cette valeur de la résistivité sera comparée à une valeur calibrée en fonction du seuil de détection retenu qui est enregistré dans le système électronique.

L'analyse de cette comparaison pourra alors déclencher une alarme par exemple visuelle (diode) ou sonore (buzzer) ou le changement d'état d'un bit si le système électronique intègre un microprocesseur.

L'alarme pourra par exemple se situer sur le boîtier de contrôle portable précédemment décrit.

Un autre paramètre important à évaluer dans le cadre de la thérapie de compression peut être la température.

La mesure de la température peut être effectuée de façon classique à l'aide d'un capteur de température comme une thermistance dont la résistance varie en fonction de la température.

De tels capteurs de température sont bien connus de l'homme du métier et utilisés dans le domaine textile.

Le système de mesure de pression d'interface selon la présente invention pourra être de formes et de configurations variées.

Dans le cadre de la présente invention, et en particulier pour le traitement des ulcères de jambe par thérapie compressive, on préfère deux formes particulières illustrées sur les figures 6 et 7.

La première forme dite du type « chaussette » est représentée schématiquement sur la figure 7.

Le support S comprend :
- une première partie S1 s'étendant sensiblement longitudinalement et délimitée par les deux capteurs de pression P₁ et P₂ destinés à être positionnés sur les points B₁ et C (voir figure 1) par exemple sur la face externe de la jambe ; et
- une deuxième partie S2 prolongeant la première partie sensiblement perpendiculairement et qui comprend les deux électrodes H formant le détecteur d'humidité, laquelle deuxième partie est destinée à être rabattue en position d'utilisation à l'intérieur de la jambe pour recouvrir la surface supérieure du pansement qui est positionné sur l'ulcère de jambe.

Dans l'exemple représenté, les composants du dispositif embarqué (source d'énergie, module de communication, antenne, microprocesseur) sont rassemblés sur un circuit imprimé unique E disposé entre les deux capteurs P₁ et P₂ .Cette disposition est particulièrement avantageuse puisqu'elle favorise au maximum, lors de l'extension du support S, la conservation de l'intégrité des pistes conductrices 1, 1A et 2, 2A qui desservent les capteurs P₁ et P₂, ainsi que celle des pistes 3 et 4 qui desservent les électrodes H.

Cette forme « chaussette » nécessite la réalisation de deux versions distinctes (gauche et droite) en fonction de la jambe à soigner.

La seconde forme est représentée schématiquement sur la figure 6.

Les deux parties S1 et S2 formant le support sont ici alignées de sorte que cette forme unique convient quel que soit la jambe (gauche ou droite) à traiter.

Le support peut être replié au niveau de la zone séparant ses deux parties constitutives S1 et S2 en permettant ainsi de positionner le détecteur d'humidité, lors de l'utilisation, du côté gauche ou droit en fonction de la jambe traitée (voir figure 1A). Le repli peut donc être avantageusement dimensionné en fonction de la taille de jambe du patient et de la localisation de la plaie.

De même, la longueur des électrodes formant le dispositif de mesure d'humidité permet de mesurer si nécessaire l'excès d'humidité sur la surface externe du pansement et/ou à la périphérie de ce dernier en cas de relargage des exsudats par les bords du pansement.

Bien entendu, le détecteur d'humidité pourra être remplacé par un capteur de température ou tout autre type de capteur.

Selon une variante de réalisation, on pourra disposer sur la deuxième partie S2 du support un détecteur d'humidité et un capteur de température.

Selon une autre variante de réalisation, on pourra disposer un détecteur d'humidité sur la deuxième partie S2 du support et un capteur de température sur une troisième partie du support (non représentée) prolongeant la première partie S1 du support du coté opposé à la deuxième partie.

Comme indiqué précédemment, les deuxième et troisième parties du support pourront être extensibles ou non. Cependant, pour faciliter la fabrication, on préférera utiliser un support extensible sur toute sa longueur.

Le système de mesure de pression d'interface selon l'invention, pourra être fixé sur le membre à traiter à l'aide de tout moyen connu de fixation d'un dispositif sur le corps humain tels qu'une fixation de type Velcro, ou un adhésif.

Dans le cadre du traitement par thérapie compressive, on préférera tout particulièrement un adhésif atraumatique si nécessaire repositionnable de la même nature que ceux qui sont couramment employés pour la réalisation des pansements absorbants destinés à être positionnés sur la peau ou sur les ulcères de jambe. Cet adhésif pourra par exemple recouvrir de façon continue ou discontinue la surface du support du système qui vient en contact avec la peau ou la plaie. On pourra se référer par exemple à la demande de brevet WO 2006/094513 pour le choix d'un tel adhésif. Si nécessaire, cet adhésif pourra servir pour encapsuler tout ou partie des composants du système de mesure de pression d'interface.

## Revendications

1. Système de mesure de pression d'interface exercée sur la peau, utile en thérapie compressive, comprenant
- un support (S) portant des capteurs dont au moins deux capteurs de pression (P₁, P₂) disposés de façon espacée sur le support pour permettre de mesurer la pression s'exerçant en deux emplacements dont l'un (B1) est prédéterminé ;
- un système électronique relié aux capteurs et apte à acquérir les valeurs mesurées simultanément par ces capteurs (P₁, P₂) ; chaque capteur de pression (P₁, P₂) comprenant un élément transducteur, de préférence sensiblement plat,
**caractérisé en ce que** ledit élément transducteur présente une surface recouverte d'une couche intermédiaire destinée à venir au contact de la peau en position d'utilisation ; ladite couche intermédiaire présentant un module élastique de compression, mesuré au niveau de la surface de ladite couche intermédiaire destinée à venir au contact de la peau, compris entre 30 et 500 kPa, de préférence entre 80 et 400 kPa et de préférence encore entre 200 et 400 kPa.

2. Système de mesure selon la revendication 1, **caractérisé en ce que** ledit matériau constituant ladite couche intermédiaire est choisi parmi :
- les matériaux alvéolaires, tels qu'en particulier des mousses, de préférence hydrophobes, comme par exemple des mousses en polyéthylène réticulé, en polyuréthane, en silicone, en polychlorure de vinyle, en polyéthylène-propylène diène, en néoprène ;
- les gels, de préférence hydrophobes, comme par exemple des gels de silicone ou de polyuréthane ;
- les mélanges à base de polymères thermoplastiques de préférence hydrophobes, comme par exemple des mélanges de polymères tri-blocs et de plastifiant.

3. Système de mesure selon l'une des revendications 1 ou 2, **caractérisé en ce que** chaque capteur de pression (P₁, P₂) est sensiblement plat et choisi parmi les capteurs résistifs, les capteurs piézo-électriques, les capteurs magnétiques à effet Hall, les capteurs capacitifs.

4. Système de mesure selon l'une quelconque des revendications 1 à 3, **caractérisé en ce que** le système électronique précité comprend :
- un dispositif embarqué (10) relié au support (S), de préférence en y étant fixé, et permettant l'acquisition et le traitement des valeurs mesurées par les capteurs de pression (P₁, P₂), ledit dispositif comportant des moyens de transmission (12) desdites valeurs mesurées par les capteurs (P₁, P₂) ;
- un dispositif déporté (20) comportant :
- des moyens de communication (23) compatibles avec les moyens de transmission (12) dudit dispositif embarqué (10) et assurant au moins la transmission des données du dispositif embarqué (10) vers le dispositif déporté (20) ; et
- un circuit de traitement (21) relié à un dispositif d'affichage (24).

5. Système de mesure selon l'une des revendications 1 à 4, **caractérisé en ce que** le support (S) précité est formé d'un matériau choisi parmi les matériaux textiles, les matériaux alvéolaires, les matériaux sous forme de films et les complexes associant deux de ces matériaux.

6. Système de mesure selon l'une des revendications 1 à 5, **caractérisé en ce que** le support (S) est non occlusif et présente, de préférence, une perméabilité à la vapeur d'eau, mesurée selon la norme EN-13726, supérieure ou égale à 1000 g/m²/24 h.

7. Système de mesure selon la revendication 6, **caractérisé en ce que** le support (S) est en non tissé ou un film en polyuréthane.

8. Système de mesure selon l'une des revendications 1 à 7, **caractérisé en ce que** le support précité est extensible et présente de préférence une extensibilité de l'ordre de 10 à 20 % dans le sens longitudinal et de l'ordre de 3 à 12 % dans le sens transversal.

9. Système de mesure selon l'une des revendications 1 à 8, **caractérisé en ce que** le support précité porte en outre :
- au moins un capteur d'humidité (H) et/ou
- au moins un capteur de température.

10. Système de mesure selon l'une des revendications 4 à 9, **caractérisé en ce que** le dispositif embarqué (10) précité est fixé au support précité et disposé entre les deux capteurs de pression (P₁, P₂).

11. Système de mesure selon la revendication 9 ou 10, **caractérisé en ce que** le capteur d'humidité (H) et/ou le capteur de température précités sont disposés sur le support (S) extérieurement à l'espace défini entre les deux capteurs de pression (P₁, P₂), de préférence dans une partie prolongeant le support dans l'axe défini par les capteurs de pression ou dans un axe perpendiculaire à celui-ci.

12. Kit de contention, **caractérisé en ce qu'**il comprend :
- un système de contention ; et
- un système de mesure de pression d'interface selon l'une quelconque des revendications 1 à 11.

## Patentansprüche

1. System zur Messung des Schnittstellendrucks, der auf die Haut ausgeübt wird, das bei der Kompressionstherapie anwendbar ist, umfassend
- einen Träger (S), der Sensoren trägt, darunter zumindest zwei Drucksensoren (P₁, P₂), die auf beabstandete Weise auf dem Träger angeordnet sind, um zu erlauben, den Druck zu messen, der an zwei Stellen ausgeübt wird, von welchen eine (B1) vorbestimmt ist,
- ein elektronisches System, das mit den Sensoren verbunden und dazu geeignet ist, die gleichzeitig von diesen Sensoren (P₁, P₂) gemessenen Werte zu erfassen, wobei jeder Drucksensor (P₁, P₂) ein Messwandlerelement umfasst, das vorzugsweise im Wesentlichen flach ist, **dadurch gekennzeichnet, dass** das Messwandlerelement eine Oberfläche aufweist, die von einer Zwischenschicht bedeckt ist, die dazu bestimmt ist, in der Verwendungsposition mit der Haut in Kontakt zu gelangen, wobei die Zwischenschicht einen Elastizitätsmodul aufweist, der an der Oberfläche der Zwischenschicht, die dazu bestimmt ist, mit der Haut in Kontakt zu gelangen, gemessen zwischen 30 und 500 kPa, vorzugsweise zwischen 80 und 400 kPa und noch bevorzugter zwischen 200 und 400 kPa beträgt.

2. System zur Messung nach Anspruch 1, **dadurch gekennzeichnet, dass** das Material, das die Zwischenschicht bildet, ausgewählt ist aus:
- den zelligen Materialien, wie insbesondere vorzugsweise hydrophoben Schäumen, wie zum Beispiel Schäumen aus vernetztem Polyethylen, aus Polyurethan, aus Silikon, aus Polyvinylchlorid, aus Polyethylen-Propylen-Dien, aus Neopren,
- den vorzugsweise hydrophoben Gelen, wie zum Beispiel Silikon- oder Polyurethangelen,
- den Mischungen auf Basis von vorzugsweise hydrophoben thermoplastischen Polymeren, wie zum Beispiel Mischungen aus Triblockpolymeren und Weichmacher.

3. System zur Messung nach einem der Ansprüche 1 oder 2, **dadurch gekennzeichnet, dass** jeder Drucksensor (P₁, P₂) im Wesentlichen flach und aus den Widerstandssensoren, den piezoelektrischen Sensoren, den Halleffekt-Magnetsensoren und den kapazitiven Sensoren ausgewählt ist.

4. System zur Messung nach einem der Ansprüche 1 bis 3, **dadurch gekennzeichnet, dass** das vorerwähnte elektronische System umfasst:
- eine mitgeführte Vorrichtung (10), die mit dem Träger (S) verbunden ist, vorzugsweise indem sie daran fixiert ist, und die Erfassung und die Verarbeitung der von den Drucksensoren (P₁, P₂) gemessenen Werte erlaubt, wobei die Vorrichtung Mittel zur Übertragung (12) der von den Sensoren (P₁, P₂) gemessenen Werte beinhaltet,
- eine entfernt befindliche Vorrichtung (20), die beinhaltet:
- Mittel zur Kommunikation (23), die mit den Mitteln zur Übertragung (12) der mitgeführten Vorrichtung (10) kompatibel sind und zumindest die Übertragung von Daten der mitgeführten Vorrichtung (10) an die entfernt befindliche Vorrichtung (20) sicherstellen, und
- eine Verarbeitungsschaltung (21), die mit einer Anzeigevorrichtung (24) verbunden ist.

5. System zur Messung nach einem der Ansprüche 1 bis 4, **dadurch gekennzeichnet, dass** der vorerwähnte Träger (S) aus einem Material gebildet ist, das aus den textilen Materialien, den zelligen Materialien, den Materialien in Form von Folien und den Verbundmaterialien, die zwei dieser Materialien kombinieren, ausgewählt ist.

6. System zur Messung nach einem der Ansprüche 1 bis 5, **dadurch gekennzeichnet, dass** der Träger (S) keinen dichten Verschluss bildet und vorzugsweise eine Dampfdurchlässigkeit, gemessen nach der Norm EN-13726, von mehr als oder gleich 1.000 g/m²/24 h aufweist.

7. System zur Messung nach Anspruch 6, **dadurch gekennzeichnet, dass** der Träger (S) aus einem Vlies oder einem Film aus Polyurethan besteht.

8. System zur Messung nach einem der Ansprüche 1 bis 7, **dadurch gekennzeichnet, dass** der vorerwähnte Träger dehnbar ist und vorzugsweise eine Dehnbarkeit in der Größenordnung von 10 bis 20 % in der Längsrichtung und in der Größenordnung von 3 bis 12 % in der Querrichtung aufweist.

9. System zur Messung nach einem der Ansprüche 1 bis 8, **dadurch gekennzeichnet, dass** der vorerwähnte Träger ferner trägt:
- zumindest einen Feuchtigkeitssensor (H) und/oder
- zumindest einen Temperatursensor.

10. System zur Messung nach einem der Ansprüche 4 bis 9, **dadurch gekennzeichnet, dass** die vorerwähnte mitgeführte Vorrichtung (10) auf dem vorerwähnten Träger fixiert und zwischen den zwei Drucksensoren (P₁, P₂) angeordnet ist.

11. System zur Messung nach Anspruch 9 oder 10, **dadurch gekennzeichnet, dass** der vorerwähnte Feuchtigkeitssensor (H) und/oder der vorerwähnte Temperatursensor auf dem Träger (S) außerhalb des Raums, der zwischen den zwei Drucksensoren (P₁, P₂) definiert ist, angeordnet ist/sind, vorzugsweise in einem Teil, der den Träger in der Achse, die durch die Drucksensoren definiert ist, oder in einer Achse senkrecht auf diese verlängert.

12. Fixier- und Stützkit, **dadurch gekennzeichnet, dass** es umfasst:
- ein Fixier- und Stützsystem und
- ein System zur Messung des Schnittstellendrucks nach einem der Ansprüche 1 bis 11.

## Claims

1. A system for measuring the interface pressure exerted on the skin, useful in compression therapy, comprising:
- a supporting member (S) bearing sensors, including at least two pressure sensors (P₁, P₂) placed apart on the supporting member, so as to be able to measure the pressure being exerted at two locations, one of which (B₁) is predetermined; and
- an electronic system connected to the sensors and capable of acquiring the values simultaneously measured by these sensors (P₁, P₂);
each pressure sensor (P₁, P₂) comprising a transducer element, which is preferably substantially flat **characterized in that** said transducer element has a surface covered by an intermediate layer intended to come into contact with the skin in the use position, said intermediate layer having a compressive elastic modulus, measured at the surface of said intermediate layer intended to come into contact with the skin, comprised between 30 and 500 kPa, preferably between 80 and 400 kPa and more preferably between 200 and 400 kPa.

2. The measurement system as claimed in claim 1, **characterized in that** said material constituting said intermediate layer is chosen from:
- cellular materials, such as in particular foams, preferably hydrophobic foams, such as for example foams made of crosslinked polyethylene, polyurethane, silicone, polyvinyl chloride, ethylene-propylene diene copolymer or neoprene;
- gels, preferably hydrophobic gels, such as for example silicone gels or polyurethane gels; and
- mixturess based on thermoplastic, preferably hydrophobic, polymers, such as for example mixtures consisting of triblock polymers and a plasticizer.

3. The measurement system as claimed in either of claims 1 and 2, **characterized in that** each pressure sensor (P₁, P₂) is substantially flat and chosen from resistive sensors, piezoelectric sensors, Hall-effect magnetic sensors and capacitive sensors.

4. The measurement system as claimed in any one of claims 1 to 3, **characterized in that** the aforementioned electronic system comprises:
- an onboard device (10) connected to the supporting member (S), preferably by being fixed thereto, and serving for the acquisition and processing of the values measured by the pressure sensors (P₁, P₂), said device comprising means (12) for transmitting said values measured by the sensors (P₁, P₂); and
- a remote device (20) comprising:
- communication means (23) compatible with the transmission means (12) of the onboard device (10) and at least transmitting the data from the onboard device (10) to the remote device (20) and
- a processing circuit (21) connected to a display device (24).

5. The measurement system as claimed in one of claims 1 to 4, **characterized in that** the aforementioned supporting member (S) is formed from a material chosen from fabric materials, cellular materials, materials in film form and complexes combining two of these materials.

6. The measurement system as claimed in one of claims 1 to 5, **characterized in that** the supporting member (S) is nonocclusive and preferably has a water vapor permeability, measured according to the EN-13726 standard, equal to or greater than 1000 g/m²/24 h.

7. The measurement system as claimed in claim 6, **characterized in that** the supporting member (S) is made of a nonwoven or a polyurethane film.

8. The measurement system as claimed in one of claims 1 to 7, **characterized in that** the aforementioned supporting member is extensible and preferably has an extensibility of around 10 to 20% in the longitudinal direction and around 3 to 12% in the transverse direction.

9. The measurement system as claimed in one of claims 1 to 8, **characterized in that** the aforementioned supporting member further bears:
- at least one moisture sensor (H); and/or
- at least one temperature sensor.

10. The measurement system as claimed in one of claims 4 to 9, **characterized in that** the aforementioned onboard device (10) is fixed to the aforementioned supporting member and placed between the two pressure sensors (P₁, P₂).

11. The measurement system as claimed in claim 9 or 10, **characterized in that** the aforementioned moisture sensor (H) and/or temperature sensor are/is placed on the supporting member (S) external to the space defined between the two pressure sensors (P₁, P₂), preferably in a part extending the supporting member along the axis defined by the pressure sensors or along an axis perpendicular thereto.

12. A restraint kit, **characterized in that** it comprises:
- a restraint system; and
- an interface pressure measurement system as claimed in any one of claims 1 to 11.
